# EUROPÄISCHE PATENTANMELDUNG

(11) **EP 4 621 390 A1**
(43) Veröffentlichungstag der Anmeldung: **24.09.2025**
(21) Anmeldenummer: 24164036.6
(22) Anmeldetag: 18.03.2024
(51) Int. Cl.: G01N 21/65, G01J 3/44, G01N 33/18, G01N 33/44, G01N 15/0205, G01N 15/1434, G01N 21/53, G01N 15/14, G01N 15/00

(54) **ANORDNUNG UND VERFAHREN ZUR VERMESSUNG VON PARTIKELN, INSBESONDERE MIKROPLASTIKPARTIKELN, IN FLÜSSIGKEITEN**

(71) Anmelder: Deckma Hamburg GmbH, 22525 Hamburg (DE)
(72) Erfinder: Nieder, Timo, 22527 Hamburg (DE); Schmidt, Axel André, 22587 Hamburg (DE); Offermann, Jochen, 21502 Geesthacht (DE)
(74) Vertreter: Glawe, Delfs, Moll

(57) **Zusammenfassung**

Anordnung zur Vermessung von in einer Flüssigkeit mitgeführten Partikeln (20), insbesondere Mikroplastikpartikeln. Die Anordnung umfasst:
einen Messkanal (14) mit einem Eingang zum Zuführen der Flüssigkeit; eine Beleuchtungseinheit (13) zur Aussendung eines Messstrahlengangs (16), der auf ein im Messkanal (14) befindliches Messvolumen (19) der Flüssigkeit auftrifft und wenigstens einen im Messvolumen (19) befindlichen Partikel (20) zur Aussendung von Streulicht anregt; ein Spektrometer (18) zur Erfassung eines inelastischen Streulichtspektrums des ausgesendeten Streulichts; eine Detektionsoptik (15), die dazu eingerichtet ist, Anteile (16a, 16b) des Streulichts, die entlang einer Detektionsrichtung auf die Detektionsoptik (15) treffen, zum Spektrometer (18) weiterzuleiten; und eine Auswerteeinheit (21) zur Identifizierung wenigstens einer Materialart des im Messvolumen (19) befindlichen Partikels (20) anhand des vom Spektrometer (18) aufgenommenen Streulichtspektrums. Erfindungsgemäß liegt ein Winkel zwischen einer optischen Achse des Messstrahlengangs (16) und der Detektionsrichtung in einem Bereich zwischen 45° und 135°. Durch die Ausrichtung der Detektionsrichtung relativ zum Messstrahlengang wird die Signalqualität verbessert.

## Beschreibung

Die Erfindung betrifft eine Anordnung sowie ein Verfahren zur Vermessung von Partikeln, insbesondere von Mikroplastikpartikeln, in Flüssigkeiten.

Der Eintrag von Mikroplastik in die Umwelt steht zunehmend im Fokus, da immer mehr negative Einflüsse der Mikroplastikbelastung bekannt werden. Um das Ausmaß der Belastung feststellen zu können, sind geeignete Laborverfahren zur Analyse des Mikroplastiks in Flüssigkeiten erforderlich. Bisher bekannte Laborverfahren sind jedoch aufwendig, zeitintensiv und ermöglichen pro Zeiteinheit nur die Vermessung einer vergleichsweise geringen Flüssigkeitsmenge.

Vor diesem Hintergrund ist es die Aufgabe der vorliegenden Erfindung, eine Anordnung sowie ein Verfahren zur Vermessung von Partikeln, insbesondere von Mikroplastikpartikeln, in Flüssigkeiten bereitzustellen, die bzw. das pro Zeiteinheit eine Vermessung größerer Flüssigkeitsmengen ermöglicht. Gelöst wird diese Aufgabe mit den Merkmalen der unabhängigen Ansprüche. Vorteilhafte Ausführungsformen sind in den Unteransprüchen angegeben.

Demnach betrifft die Erfindung eine Anordnung zur Vermessung von in einer Flüssigkeit mitgeführten Partikeln, insbesondere Mikroplastikpartikeln, wobei die Anordnung umfasst: einen Messkanal mit einem Eingang zum Zuführen der Flüssigkeit; eine Beleuchtungseinheit zur Aussendung eines Messstrahlengangs, der auf ein im Messkanal befindliches Messvolumen der Flüssigkeit auftrifft und wenigstens einen im Messvolumen befindlichen Partikel zur Aussendung von Streulicht anregt; ein Spektrometer zur Erfassung eines inelastischen Streulichtspektrums des ausgesendeten Streulichts; eine Detektionsoptik, die dazu eingerichtet ist, Anteile des Streulichts, die entlang einer Detektionsrichtung auf die Detektionsoptik treffen, zum Spektrometer weiterzuleiten; und eine Auswerteeinheit zur Identifizierung wenigstens einer Materialart des im Messvolumen befindlichen Partikels anhand des vom Spektrometer aufgenommenen Streulichtspektrums, wobei ein Winkel zwischen einer optischen Achse des Messstrahlengangs und der Detektionsrichtung in einem Bereich zwischen 45° und 135° liegt.

Zunächst werden einige im Rahmen der Erfindung verwendete Begriffe erläutert. Die Verwendung eines inelastischen Streulichtspektrums zur Identifizierung eines Materials ist aus dem Stand der Technik grundsätzlich bekannt. Bei der inelastischen Lichtstreuung, die auch als Raman-Streuung bezeichnet werden kann, trifft ein Lichtteilchen auf ein Streuzentrum, wobei das Streuzentrum anschließend ein Lichtteilchen emittiert, dessen Energie sich von dem einfallenden Lichtteilchen unterscheidet. Der Unterschied rührt daher, dass eine Energieübertragung zwischen dem anregenden Lichtteilchen und der angeregten Materie stattfindet, wobei das Lichtteilchen dabei Energie an die Materie abgeben kann (sogenannte Stokes-Raman-Streuung) oder aber Energie von der Materie aufnehmen kann (sogenannte Anti-Stokes-Raman-Streuung). Die Energieaufnahme bzw. Abgabe innerhalb der Materie führt zu einer entsprechenden Änderung der Schwingungs- oder Rotationsenergie der innerhalb der Materie befindlichen Moleküle, wobei das Ausmaß der Energiedifferenz spezifisch für die beteiligten Moleküle ist. Durch eine Vermessung der Wellenlängen des emittierten inelastisch gestreuten Lichts (also des Stokes-Raman-Spektrums und/oder des Anti-Stokes-Raman-Spektrums) kann daher ein Rückschluss auf die untersuchte Materie gezogen werden. Die erfindungsgemäße Anordnung eignet sich besonders zur Vermessung von Mikroplastikpartikeln, ist jedoch nicht darauf beschränkt und eignet sich auch zur Vermessung von Partikeln, die aus anderen Materialien bestehen, oder beispielsweise auch zur Vermessung von in der Flüssigkeit befindlichen Tropfen anderer Flüssigkeiten (beispielsweise Öl oder flüssige Polymere). Der Begriff "Partikel" ist somit weit zu verstehen und umfasst insbesondere auch in der Flüssigkeit mitgeführte Flüssigkeitspartikel.

Der auf das Messvolumen auftreffende Messstrahlengang eignet sich zur Anregung eines inelastischen Streulichtspektrums, das eine Identifizierung der Materialart des im Messvolumen befindlichen Partikels ermöglicht. Der Messstrahlengang kann insbesondere durch Licht einer geringen spektralen Breite gebildet sein, die beispielsweise weniger als 30 nm, insbesondere weniger als 15 nm, weiter insbesondere weniger als 5 nm betragen kann. Darüber hinaus kann der Messstrahlengang kohärentes Licht umfassen oder daraus gebildet sein. Die Beleuchtungseinheit kann zur Erzeugung des Messstrahlengangs eine Laserlichtquelle aufweisen. Eine mittlere Wellenlänge des Messstrahlengangs kann beispielsweise bei 635 nm liegen. Die Anordnung kann eine Fördereinrichtung zum Zuführen der Flüssigkeit zum Eingang des Messkanals aufweisen. Der Messkanal kann sich entlang einer Strömungsrichtung von dem Eingang zu einem Ausgang zum Abführen der Flüssigkeit erstrecken. In diesem Fall kann vorgesehen sein, dass dem Messkanal die Flüssigkeit kontinuierlich zugeführt wird.

Aus dem Stand der Technik war es grundsätzlich bekannt (vgl. CN 113176248 A), zur Vermessung von Mikroplastik-Partikeln eine Raman-Messung durchzuführen, bei der ein Streulichtspektrum analysiert wird, das in 180°-Rückstreuung erfasst wurde. Die Erfassung in 180°-Rückstreuung ist bei Raman-Messungen üblich, da dabei für den anregenden Messstrahl und das Streulicht dieselbe Optik verwendet werden kann. Im Rahmen der Erfindung wird hingegen vorgeschlagen, dass die optische Achse des Messstrahlengangs und die Detektionsrichtung in einem Bereich zwischen 45° und 135° liegen. Es wurde erkannt, dass dadurch die Signalqualität gegenüber der Messung bei 180°-Rückstreuung deutlich verbessert werden kann. Insbesondere wird der Anteil des elastischen Streulichts, das für die spätere Auswertung des inelastischen Streulichtspektrums störend ist, bei Detektion im genannten Winkelbereich deutlich reduziert und so die Qualität des Messignals verbessert. Ein zur Erfassung eines ausreichenden Messsignals erforderlicher Messzeitraum kann auf diese Weise deutlich verringert werden, so dass pro Zeiteinheit entsprechend größere Flüssigkeitsmengen vermessen werden können.

In einer Ausführungsform liegt der Winkel zwischen der optischen Achse des Messstrahlengangs und der Detektionsrichtung in einem Bereich zwischen 60° und 120°, insbesondere zwischen 75° und 105°. Dadurch können durch das elastische Streulicht erzeugte Störsignale weiter reduziert werden.

Es kann vorgesehen sein, dass die auf die Detektionsoptik auftreffenden Anteile des Streulichts einen ersten Anteil des Streulichts umfassen, der ausgehend vom Partikel auf direktem Weg auf die Detektionsoptik trifft, wobei die Anordnung weiterhin eine Reflexionsoptik umfasst, die dazu eingerichtet ist, einen zweiten vom ersten verschiedenen Anteil des Streulichts durch Reflexion entlang der Detektionsrichtung zur Detektionsoptik umzulenken, so dass die Detektionsoptik den zweiten Anteil des Streulichts zum Spektrometer weiterleitet. Durch die Reflexionsoptik kann somit zusätzlich zu dem Streulicht, das ausgehend vom Partikel auf direktem Weg auf die Detektionsoptik trifft, ein weiterer Anteil des Streulichts in die Detektionsoptik umgelenkt und von dort aus zur Erzeugung des Streulichtspektrums an das Spektrometer weitergeleitet werden. Die Ausbeute des an das Spektrometer weitergeleiteten inelastischen Streulichts kann auf diese Weise erhöht und das Messsignal weiter verbessert werden.

In einer Ausführungsform weist das Spektrometer einen Detektor mit einer Mehrzahl von entlang einer Transversalrichtung nebeneinander positionierten Detektorchips auf. Das Spektrometer kann dazu eingerichtet sein, verschiedene Wellenlängenbereiche des empfangenen Streulichts in unterschiedliche Winkelbereiche aufzufächern, so dass die verschiedenen Wellenlängenbereiche auf verschiedene der Mehrzahl von Detektorchips gelenkt werden. Zur Auffächerung des inelastischen Streulichts in unterschiedliche Winkelbereiche kann das Spektrometer beispielsweise ein optisches Gitter umfassen. Weiterhin kann vorgesehen sein, dass jeder Detektorchip einen Signalausgang und jeweils eine Vielzahl von mit dem Signalausgang verbundenen Photoelektronenvervielfacher-Zellen, insbesondere Lawinenphotodioden aufweist. Darüber hinaus kann eine in Richtung der Transversalrichtung gemessene Seitenlänge der Detektorchips zwischen 0,2 mm und 2 mm, vorzugsweise zwischen 0,4 mm und 1,5 mm, weiter vorzugsweise zwischen 0,7 mm und 1,3 mm liegen. Photoelektronenvervielfacher-Zellen bzw. Lawinenphotodioden zeichnen sich dadurch aus, dass sie eine sehr hohe Sensitivität aufweisen und dass üblicherweise bereits bei Auftreffen eines einzelnen Lichtteilchens auf eine der Zellen durch den "Lawineneffekt" ein großes elektrisches Signal erzeugt wird. Indem eine Vielzahl solcher Zellen zu einem großflächigen Detektorchip mit einem einzelnen Signalausgang zusammengefasst werden, können Lichtteilchen mit äußerst hoher Sensitivität erfasst werden, wobei der Detektorchip gleichzeitig eine hohe Dynamik aufweist. Insbesondere kann jede der Vielzahl von Zellen nach dem Auftreffen eines Lichtteilchens eine elektrische Ladung an den gemeinsamen Signalausgang weiterleiten, wobei die elektrischen Ladungen über den Signalausgang akkumuliert bzw. integriert werden können, um eine Gesamtintensität zu erfassen. Die Vielzahl von mit dem Signalausgang verbundenen Photoelektronenvervielfacher-Zellen kann durch eine Anzahl von Photoelektronenvervielfacher-Zellen gebildet sein, die im Bereich zwischen 1000 und 20000, vorzugsweise zwischen 2000 und 15000, weiter vorzugsweise zwischen 3000 und 10000 liegt. Eine Fläche einer einzelnen Photoelektronenvervielfacher-Zelle kann eine Seitenlänge aufweisen, die zwischen 10 µm und 100 µm liegt. Trifft ein Lichtteilchen auf eine einzelne der Vielzahl von Zellen, ist diese für eine gewisse Totzeit für die Erfassung weiterer Lichtteilchen nicht mehr empfänglich. Weitere auf den Detektorchip treffende Lichtteilchen können jedoch weiterhin von den anderen der Vielzahl von Zellen detektiert werden, wodurch eine hohe Dynamik erreicht wird.

Durch die vorstehend genannte große Anzahl von Photoelektronenvervielfacher-Zellen sowie der damit einhergehenden großen Fläche der Detektorchips reduziert sich hingegen die erzielbare Wellenlängenauflösung. Es wurde allerdings erkannt, dass die Vorteile der hohen Sensitivität und Dynamik die Nachteile der geringen Auflösung für den vorliegenden Messzweck mehr als kompensieren. Insbesondere kann aufgrund der hohen Sensitivität eine äußerst geringe Integrationszeit (also eine Zeit innerhalb derer ein Detektorchip die am gemeinsamen Signalausgang ankommenden Ladungen integriert) verwendet werden. Eine Integrationszeit, die für eine aussagekräftige Messung ausreichend ist, kann beispielsweise weniger als 50 ms, insbesondere weniger als 25 ms, weiter insbesondere weniger als 15 ms betragen. Vorstehend genannte Integrationszeiten sind deutlich kleiner, als es im Stand der Technik bei der Vermessung von inelastischem Streulicht mit gewöhnlichen CCD-Sensoren üblich ist.

Es kann vorgesehen sein, dass die Mehrzahl von entlang der Transversalrichtung nebeneinander positionierten Detektorchips durch eine Anzahl von Detektorchips gebildet ist, die im Bereich zwischen 30 und 300, vorzugsweise zwischen 40 und 200, weiter vorzugsweise zwischen 50 und 150 liegt. Es hat sich gezeigt, dass durch die genannte Anzahl von Detektorchips ein ausreichend breiter Spektralbereich mit einer ausreichenden Auflösung vermessen werden kann. Die Breite des mit dem Spektrometer messbaren Spektralbereichs kann beispielsweise zwischen 80 nm und 400 nm, vorzugsweise zwischen 100 nm und 300 nm liegen.

Die Detektorchips können eine senkrecht zur Seitenlänge gemessene Höhe aufweisen, die größer oder gleich das 0,5-fache und kleiner oder gleich das 2-fache, insbesondere größer oder gleich das 0,8-fache und kleiner oder gleich das 1,2-fache der Seitenlänge der Detektorchips beträgt. Wenn die Höhe der Detektorchips sich nur wenig von der Seitenlänge der Detektorchips entlang der Transversalrichtung unterscheidet, ist es möglich, einen besonders großen Anteil der Streulichtintensität auf die Detektorchips zu leiten, wobei insbesondere auf eine Spalt-Blende am Eingang des Spektrometers verzichtet werden kann.

Es kann vorgesehen sein, dass die Anordnung weiterhin eine Einrichtung zur Erfassung einer Intensität des von der Detektionsoptik an das Spektrometer weitergeleiteten Streulichts aufweist. Die Auswerteeinheit kann zudem dazu ausgebildet sein, anhand der Intensität dieses Streulichts eine Partikelgröße zu ermitteln. Es wurde erkannt, dass die Intensität des Streulichts Rückschlüsse auf die Partikelgröße zulässt, da größere Partikel eine größere Menge an Streulicht erzeugen.

In einer Ausführungsform umfasst das Spektrometer ein optisches Gitter, wobei die Intensität des Streulichts anhand einer nullten Beugungsordnung des optischen Gitters erfasst wird und wobei das inelastische Streulichtspektrum anhand einer Beugungsordnung des optischen Gitters erfasst wird, die höher ist als die nullte Beugungsordnung, insbesondere anhand einer ersten Beugungsordnung. Bei dieser Ausführungsform wird ausgenutzt, dass bei der Beugung des Streulichts an einem optischen Gitter in der nullten Beugungsordnung sämtliche gestreuten Wellenlängen überlagert werden, so dass eine Gesamtintensität des Streulichts auf einfache Weise ermittelt werden kann, während in der ersten oder höheren Beugungsordnungen eine Wellenlängenauffächerung erfolgt, die auf die hier beschriebene Weise eine Ermittlung der Materialart ermöglicht.

Es kann vorgesehen sein, dass im Strahlengang des Spektrometers vor dem optischen Gitter ein kollimierender Spiegel und nach dem optischen Gitter ein fokussierender Spiegel positioniert ist, wobei letzterer vorzugsweise dazu eingerichtet ist, die entsprechenden Wellenlängenbereiche des Streulichts auf die jeweiligen Detektorchips zu fokussieren. Weiterhin kann vorgesehen sein, dass der Abschnitt des Strahlengangs zwischen dem kollimierenden Spiegel und dem optischen Gitter sich mit dem Abschnitt des Strahlengangs zwischen dem fokussierenden Spiegel und den Detektorchips kreuzt. Dies ermöglicht eine Verlängerung des Laufwegs des Strahlengangs, so dass die Wellenlängenbereiche räumlich weiter voneinander getrennt werden, wobei gleichzeitig die Abmessungen des Spektrometers kompakt gehalten werden. Der optische Laufweg kann in einem Bereich zwischen 10 cm und 200 cm, vorzugsweise zwischen 20 cm und 100 cm liegen.

Die Anordnung kann einen Wellenlängenfilter zur Reduzierung einer Intensität des elastischen Streulichts aufweisen. Der Wellenlängenfilter kann Teil der Detektionsoptik sein. Da der Anteil des elastischen Streulichts regelmäßig um ein Vielfaches höher ist als der Anteil des inelastischen Streulichts, kann das elastische Streulicht innerhalb des Spektrometers unerwünschte Störungen bei der Erfassung des inelastischen Streulichtspektrums bewirken. Durch den Wellenlängenfilter können diese Störungen reduziert werden. Der Wellenlängenfilter kann auf ein erwartetes Spektrum des inelastischen Streulichts abgestimmt sein. Wird beispielsweise das Stokes-Raman-Spektrum vermessen (dessen Wellenlänge größer ist als die Wellenlänge des elastischen Streulichts), kann der Wellenlängenfilter als Langpassfilter ausgebildet sein, der eine Cut-On-Wellenlänge oberhalb der Anregungswellenlänge aufweist. Entsprechend kann bei der Vermessung des Anti-Stokes-Raman-Spektrums ein Kurzpassfilter verwendet werden. Möglich ist auch die Verwendung eines band-unterdrückenden Filters, der eine Intensität eines die Anregungswellenlänge umfassenden Wellenlängenbandes auf geeignete Weise reduziert, wobei das Wellenlängenband derart gewählt ist, dass eine Intensität des Stokes-Raman-Spektrums und des Anti-Stokes-Raman-Spektrum vom Filter nicht oder nur geringfügig reduziert wird.

In einer Ausführungsform weist die Beleuchtungseinheit einen Wellenlängenfilter auf, der dazu ausgebildet ist, eine Intensität von Lichtwellenlängen, die innerhalb des zu vermessenden inelastischen Streulichtspektrums liegen, zu reduzieren. Abhängig davon, ob das zu vermessende inelastische Streulichtspektrum ein Stokes-Raman-Spektrum oder ein Anti-Stokes-Raman-Spektrum ist oder ob beide Spektren vermessen werden, kann der Filter als Kurzpassfilter, Langpassfilter oder bandunterdrückender Filter ausgebildet sein.

In einer Ausführungsform weist die Anordnung weiterhin eine Transmissionseinheit zur Erfassung einer durch das Messvolumen hindurchtretenden Transmission des Messstrahlengangs auf. Die Auswerteeinheit kann dazu eingerichtet sein, anhand der Transmission eine Anzahl und/oder eine Partikelgröße von im Messvolumen befindlichen Partikeln zu bestimmen und/oder festzustellen, ob ein im Messvolumen befindlicher Partikel sich vollständig innerhalb des Messvolumens befindet oder sich bis zu einem Rand des Messvolumens erstreckt. Die Auswerteeinheit kann beispielsweise eine Intensität der Transmission zur Bestimmung der Partikelgröße zusätzlich berücksichtigen. Möglich ist auch, dass die Transmissionseinheit einen geeigneten Sensor zur Erfassung eines Schnittbildes von im Messvolumen befindlichen Partikeln umfasst, wobei anhand des Schnittbildes ein Durchmesser ermittelt werden kann. Wenn ein Partikel bis an den Rand des Messvolumens heranreicht oder über dieses hinausragt, ist dies auf dem Sensor entsprechend ablesbar. Die anhand der Transmission gewonnenen zusätzlichen Informationen können von der Auswerteeinheit zusätzlich zum Streulicht berücksichtigt werden, um die Partikelgröße zu ermitteln.

Die Auswerteeinheit kann dazu ausgebildet sein, eine Partikelgröße des im Messvolumen befindlichen Partikels anhand des inelastischen Streulichtspektrums zu bestimmen, indem das inelastische Streulichtspektrum verglichen wird mit einem inelastischen Streulichtspektrum, das gewonnen wurde, während das Messvolumen frei von Partikeln ist. Es hat sich gezeigt, dass die Flüssigkeit (bei der es sich insbesondere um Wasser handeln kann) selbst ein spezifisches inelastisches Streulichtspektrum aufweist, das ein Intensitätsmaximum an einer charakteristischen Stelle aufweist. Im Falle von Wasser kann dieses Intensitätsmaximum bei einer Raman-Verschiebung von etwa 3400 1/cm liegen, was bei Verwendung einer Anregungswellenlänge von beispielsweise 635 nm einer Wellenlänge des Streulichts von etwa 810 nm entspricht. Es wurde erkannt, dass übliche Mikroplastikmaterialien in dieser Stelle oftmals kein Intensitätsmaximum aufweisen, so dass die Ausprägung des Intensitätsmaximums abhängig ist von der innerhalb des Messvolumens vorhandenen Flüssigkeitsmenge, die sich wiederum gegenläufig zur Partikelgröße verhält. Anhand eines Vergleichs der Höhe des für die Flüssigkeit charakteristischen Intensitätsmaximums mit der Höhe dieses Intensitätsmaximums in einem "partikelfreien" Spektrum kann daher die Partikelgröße abgeschätzt werden.

In einer Ausführungsform ist die Beleuchtungseinheit zur Aussendung eines Messstrahlengangs mit einer mittleren Strahlungsleistung ausgebildet, die größer als 0,5 W, vorzugsweise größer als 1 W, weiter vorzugsweise größer als 1,4 W ist. Darüber hinaus kann die Anordnung eine Fokussiereinheit umfassen, die dazu eingerichtet ist, den Messstrahlengang auf das Messvolumen zu fokussieren, wobei das Messvolumen im Querschnitt des Messstrahlengangs betrachtet einen Durchmesser aufweist, der zwischen 10 µm und 800 pm, vorzugsweise zwischen 20 µm und 400 µm, weiter vorzugsweise zwischen 40 µm und 160 µm liegt. Die Fokussiereinheit kann Teil der Beleuchtungseinheit sein. Durch die vorstehend genannte hohe Strahlungsleistung und die Fokussierung auf ein kleines Messvolumen, wird die Menge des erzeugten Streulichts erhöht, wodurch in kürzerer Zeit bessere Messergebnisse erhalten werden können.

Bei großer Strahlungsleistung sowie Fokussierung kann es zu einer starken Erwärmung des Messvolumens und Materialveränderungen der Probe kommen. Um eine ausreichende Kühlung zu gewährleisten, kann vorgesehen sein, dass der Messkanal einen Innendurchmesser im Bereich zwischen 3 mm und 16 mm, vorzugsweise zwischen 5 mm und 9 mm aufweist. In diesem Fall ist um das Messvolumen herum eine ausreichend große Flüssigkeitsmenge vorhanden, in die Wärmeenergie abgeleitet werden kann. Ein Außendurchmesser des Messkanals kann im Bereich zwischen 6 mm und 24 mm, vorzugsweise zwischen 8 mm und 14 mm liegen. Der Messkanal weist ein für die Anregungswellenlänge und das erwartete Streulicht transparentes Material auf und kann insbesondere ein Glasrohr umfassen.

Gegenstand der Erfindung ist weiterhin ein Verfahren zur Vermessung von in einer Flüssigkeit mitgeführten Partikeln, insbesondere Mikroplastikpartikeln, umfassend die folgenden Schritte: Zuführen der Flüssigkeit einem Messkanal,; Aussenden eines Messstrahlengangs auf ein im Messkanal befindliches Messvolumen der Flüssigkeit, um wenigstens einen im Messvolumen befindlichen Partikel zur Aussendung von Streulicht anzuregen; Weiterleiten von Anteilen des Streulichts entlang einer Detektionsrichtung zu einem Spektrometer; Erfassen eines inelastischen Streulichtspektrums des zum Spektrometer geleiteten Streulichts mit Hilfe des Spektrometers; Auswerten des erfassten Streulichtspektrums. Erfindungsgemäß liegt ein Winkel zwischen einer optischen Achse des Messstrahlengangs und der Detektionsrichtung in einem Bereich zwischen 45° und 135°. Das Auswerten kann umfassen, dass wenigstens eine Materialart des im Messvolumen befindlichen Partikels anhand des erfassten Streulichtspektrums identifiziert wird. Das Verfahren kann durch weitere Merkmale fortgebildet werden, die oben bereits in Verbindung mit der Anordnung zur Vermessung von in einer Flüssigkeit mitgeführten Partikeln beschrieben wurden. Der Messkanal kann sich entlang einer Strömungsrichtung von dem Eingang zu einem Ausgang zum Abführen der Flüssigkeit erstrecken. In diesem Fall kann vorgesehen sein, dass dem Messkanal die Flüssigkeit kontinuierlich zugeführt wird. Möglich ist alternativ auch, dass die zu vermessende Flüssigkeit durch den Eingang in den Messkanal eingeleitet wird und nach der Vermessung durch den Eingang wieder aus dem Messkanal entfernt wird.

In einer Ausführungsform umfassen die zu dem Spektrometer weitergeleiteten Anteile des Streulichts einen ersten Anteil des Streulichts, der ausgehend vom Partikel auf direktem Weg zum Spektrometer geleitet wird, wobei das Verfahren den weiteren Schritt umfasst: Reflektieren eines zweiten vom ersten verschiedenen Anteils des Streulichts, um den zweiten Anteil entlang der Detektionsrichtung zum Spektrometer weiterzuleiten.

Es kann vorgesehen sein, dass die Flüssigkeit mit einem Volumenstrom durch den Messkanal geleitet wird, der zwischen 2 ml/min und 500 ml/min, vorzugsweise zwischen 5 ml/min und 100 ml/min, weiter vorzugsweise zwischen 10 ml/min und 50 ml/min liegt. Weiterhin kann eine für den Durchfluss zur Verfügung stehende Querschnittsfläche des Messkanals zwischen 7 mm² und 200 mm², vorzugsweise zwischen 20 mm² und 60 mm² liegen. Aufgrund der oben bereits erläuterten Verkürzung des Messzeitraums, der erforderlich ist, um das inelastische Streulichtspektrum zu erfassen, kann während der Messung ein höherer, insbesondere kontinuierlicher Volumenstrom durch den Messkanal geleitet werden. Aufgrund des höheren Volumenstroms kann wiederum eine größere Wärmemenge, die durch die Beleuchtungseinheit in das Messvolumen eingebracht wird, abgeführt werden. Dies ermöglicht eine Erhöhung einer optischen Strahlungsleistung des Messstrahlengangs bzw. eine stärkere Fokussierung des Messstrahlengangs, ohne dass eine zu starke Erhitzung des Messvolumens stattfindet. Durch die höhere optische Strahlungsleistung kann das Messsignal weiter verbessert bzw. der Messzeitraum weiter verkürzt werden.

Nachfolgend werden beispielhafte Ausführungsformen der Erfindung unter Bezugnahme auf die beigefügten Zeichnungen beispielhaft erläutert. Es zeigen:
- Figur 1:: eine schematische Darstellung einer erfindungsgemäßen Anordnung zur Vermessung von in einer Flüssigkeit mitgeführten Partikeln;
- Figur 2:: eine schematische Darstellung von einigen in Figur 1 gezeigten Elementen der Anordnung in einem höheren Detailgrad;
- Figur 3:: eine schematische Darstellung der Reflexionseinheit der erfindungsgemäßen Anordnung der Figur 1 in einem größeren Detailgrad;
- Figur 4:: eine schematische Darstellung der Detektionseinheit der erfindungsgemäßen Anordnung der Figur 1 in einem größeren Detailgrad;
- Figur 5:: eine schematische Darstellung des Spektrometers der erfindungsgemäßen Anordnung der Figur 1 in einem größeren Detailgrad;
- Figur 6:: vier beispielhafte inelastische Streulichtspektren, die an drei verschiedenen Partikelmaterialien sowie an einem partikelfreien Messvolumen mit Hilfe des in Figur 5 gezeigten Spektrometers aufgenommen wurden;
- Figur 7:: sechs beispielhafte inelastische Streulichtspektren, die an fünf PMMA-Partikeln unterschiedlicher Größe sowie an einem partikelfreien Messvolumen mit Hilfe des in Figur 5 gezeigten Spektrometers aufgenommen wurden.

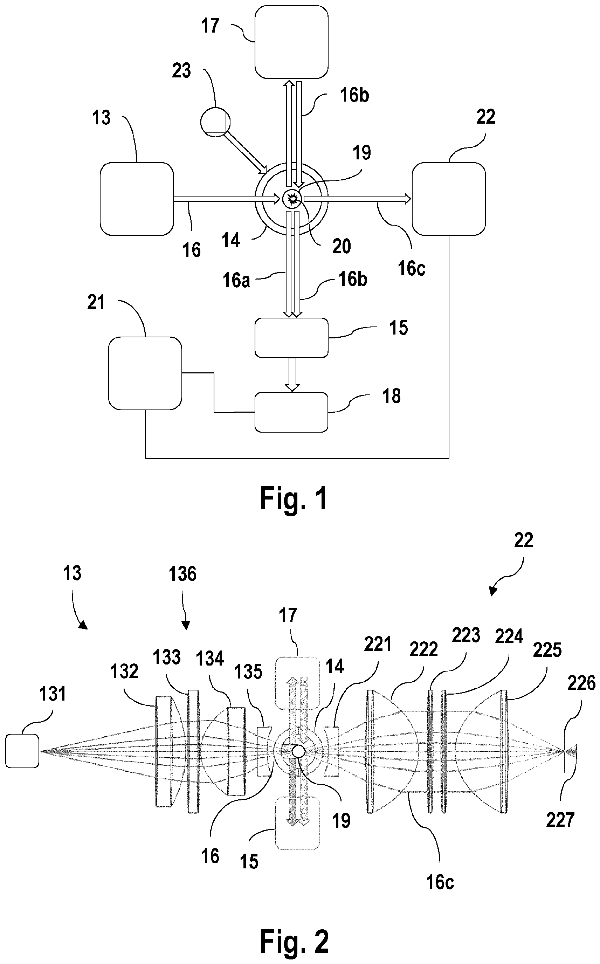

Figur 1 zeigt eine schematische Darstellung einer erfindungsgemäßen Anordnung zur Vermessung von in einer Flüssigkeit mitgeführten Partikeln. Die Anordnung umfasst eine Beleuchtungseinheit 13, die zur Aussendung eines Messstrahlengangs 16 eingerichtet ist. Der Messstrahlengang 16 trifft auf einen Messkanal, der vorliegend durch ein Glasrohr 14 mit einem Innendurchmesser von 7 mm und einem Außendurchmesser von 10 mm gebildet ist. Das Glasrohr 14 erstreckt sich vorliegend senkrecht zur Zeichenebene und ist daher in Figur 1 kreisförmig dargestellt. Das Glasrohr 14 weist einen Eingang auf (in Figur 1 nicht gezeigt), dem mit Hilfe einer Fördereinrichtung 23 eine Flüssigkeit zugeführt wird. Ein Volumenstrom durch das Glasrohr beträgt vorliegend 20 ml/min. In der Flüssigkeit sind Mikroplastikpartikel enthalten. Die Flüssigkeit strömt senkrecht zur Zeichenebene entlang einer Strömungsrichtung durch das Glasrohr 14 bis zu einem in Figur 1 nicht gezeigten Ausgang des Glasrohr 14. Eine optische Achse des Messstrahlengangs 16 ist senkrecht zur Strömungsrichtung.

Der Messstrahlengang 16 trifft auf ein innerhalb des Glasrohrs 14 befindliches Messvolumen 19. In Figur 1 ist beispielhaft ein einzelner Mikroplastikpartikel 20 dargestellt, der sich im Messvolumen 19 befindet. Der Messstrahlengang 16 wird am Partikel 20 gestreut, wobei ein erster Anteil 16a des Streulichts ausgehend vom Partikel 20 entlang einer Detektionsrichtung unmittelbar in eine Detektionsoptik 15 eintritt. Die Detektionsrichtung ist in einem 90° Winkel zu dem von der Beleuchtungseinheit 13 ausgesendeten Messstrahlengang 16 ausgerichtet. Die Detektionsoptik 15 leitet den Anteil 16a des Streulichts weiter zu einem Spektrometer 18.

Ein weiterer Anteil 16b des Streulichts gelangt ausgehend vom Partikel 20 zunächst zu einer Reflexionsoptik 17, wobei ein Winkel zwischen dem Anteil 16b und dem von der Beleuchtungseinheit 13 ausgehenden Messtrahlengang 16 270° beträgt. Die Reflexionsoptik 17 reflektiert den Anteil 16b und leitet ihn entlang der Detektionsrichtung zur Detektionsoptik 15 weiter. Auf diese Weise wird auch der reflektierte Anteil 16b des Streulichts von der Detektionsoptik 15 zum Spektrometer 18 weitergeleitet, sodass eine Streulichtausbeute deutlich erhöht wird. Die Reflexionsoptik 17 ist entlang der Detektionsrichtung auf einer gegenüberliegenden Seite des Messvolumens 19 positioniert.

Die Anordnung umfasst weiterhin eine Transmissionseinheit 22, die zur Erfassung eines Anteils 16c des Messstrahlengangs 16, der durch das Messvolumen 20 transmittiert wird (vorliegend auch als Transmission des Messstrahlengangs bezeichnet), ausgestaltet ist.

Sowohl das Spektrometer 18 als auch die Transmissionseinheit 22 sind mit einer Auswerteeinheit 21 verbunden. Die Auswerteeinheit 21 empfängt Messdaten vom Spektrometer 18 und von der Transmissionseinheit 22 und ist zur Identifizierung wenigstens einer Materialart des im Messvolumen 19 befindlichen Partikel sowie zur Bestimmung einer Partikelanzahl und einer Partikelgröße ausgestaltet.

Figur 2 zeigt eine schematische Darstellung der erfindungsgemäßen Anordnung der Figur 1, wobei die Beleuchtungseinheit 13 und die Transmissionseinheit 22 in einem höheren Detailgrad dargestellt sind und wobei andere Elemente der Übersicht halber weggelassen wurden. Die Beleuchtungseinheit 13 umfasst eine Lichtquelle 131 sowie eine aus mehreren Linsen gebildete Strahlformungsoptik 136, die aus dem Licht der Lichtquelle den Messstrahlengang 16 erzeugt. Die Lichtquelle 131 ist zur Erzeugung von Laserlicht ausgebildet, das eine Wellenlänge von 635 nm sowie eine Strahlungsleistung von 1,8 W im Continuous-Wave-Betrieb aufweist. Das Laserlicht wird über eine nicht gezeigte Lichtleitfaser abgegeben, deren Ausgang sich in einer Objektebene der Strahlformungsoptik 136 befindet. Die Strahlformungsoptik 136 umfasst eine Kollimatorlinse 132 und eine Fokuslinse 134 zur Fokussierung des Laserlichts auf das Messvolumen 19. Das Messvolumen 19 weist vorliegend im Querschnitt betrachtet einen Durchmesser von 80 µm auf. Aufgrund der innerhalb des Messvolumens 19 erzeugten hohen Energiedichte entsteht ein hinreichend intensives inelastisches Streulichtspektrum, das bereits innerhalb eines sehr kurzen Messzeitraums einen Rückschluss auf das Partikelmaterial ermöglicht. Trotz der hohen Energiedichte kann innerhalb des Messvolumens 19 eine zu starke Erwärmung der dort befindlichen Partikel verhindert werden, da das Messvolumen 19 unmittelbar von der umgebenden Flüssigkeit gekühlt und durch den im Glasrohr 14 herrschenden hohen Volumenstrom kontinuierlich weitergeführt wird, so dass die Partikel der hohen Energiedichte nur kurz ausgesetzt sind.

Die Beleuchtungseinheit 13 umfasst weiterhin eine Korrekturzylinderlinse 135, die zur Korrektur einer durch die Krümmung des Glasrohres 14 hervorgerufenen optischen Wirkung ausgebildet ist. Schließlich umfasst die Beleuchtungseinheit 13 einen Kurzpassfilter 133, der eine Cut-Off-Wellenlänge von 650 nm aufweist, also die Intensität von im Laserlicht vorhandenen Wellenlängen von größer als oder gleich 650 nm reduziert oder vollständig herausfiltert.

Der transmittierte Anteil 16c des Messstrahlengangs tritt auf der der Beleuchtungseinheit 13 gegenüberliegenden Seite des Glasrohrs 14 aus diesem aus und tritt in die Transmissionseinheit 22 ein. Die Transmissionseinheit 22 umfasst ebenfalls eine Korrekturzylinderlinse 221, die eine optische Wirkung des Glasrohrs 14 korrigiert, sowie eine aus mehreren optischen Elementen 222, 223, 224, 225 bestehende Konfokaloptik, die das Messvolumen 19 in die Ebene einer Lochblende 226 fokussiert. Hinter der Lochblende 226 befindet sich eine Photodiode 227. Mit Hilfe einer Beobachtung des auf der Photodiode 227 erzeugten Bildes des Messvolumens 19 kann die Beleuchtungseinheit 13 durch eine Translation relativ zum Glasrohr 14 korrekt ausgerichtet werden, indem der Fokuspunkt der Transmissionseinheit exakt auf die Lochblende 226 ausgerichtet und die auf die Photodiode 227 auftreffende Intensität entsprechend maximiert wird.

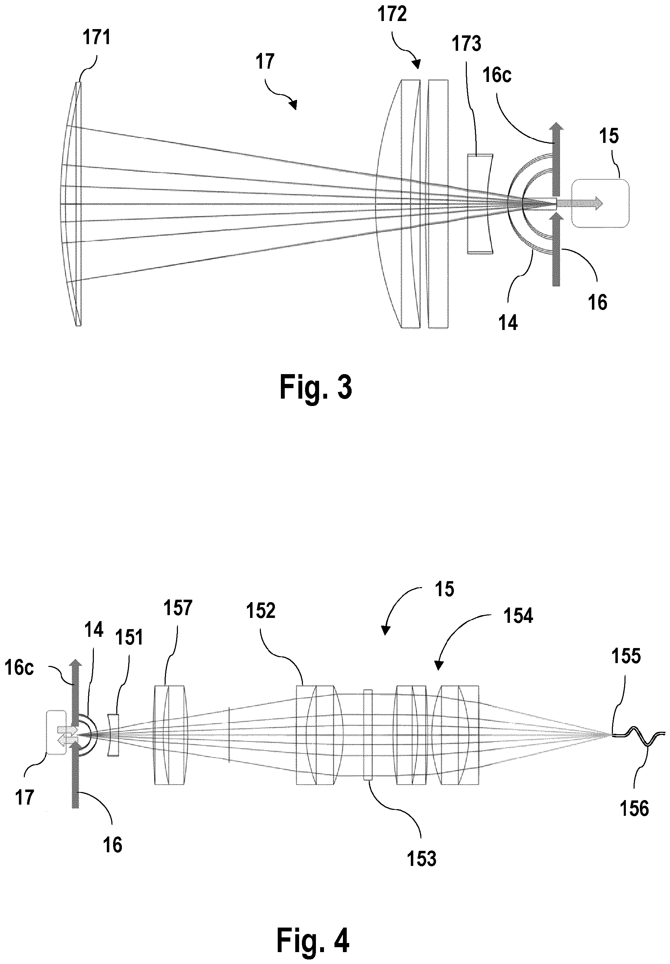

Figur 3 zeigt eine schematische Darstellung der Reflexionseinheit 17 der Figur 1 in einem größeren Detailgrad. Die Reflexionseinheit umfasst einen sphärischen Spiegel 171, Linsen 172 zur sphärischen Aberrationskorrektur und eine Korrektur Zylinderlinse 173 zur Korrektur der optischen Wirkung des Glasrohrs 14. Streulicht, das ausgehend vom Messvolumen in die Reflexionseinheit 17 eintritt, wird am sphärischen Spiegel 171 zurückgeworfen, auf das Messvolumen fokussiert und auf diese Weise in die Detektionseinheit 15 geleitet.

Figur 4 zeigt eine schematische Darstellung der Detektionseinheit 15 der erfindungsgemäßen Anordnung der Figur 1 in einem größeren Detailgrad. Die Detektionseinheit 15 nimmt, wie vorstehend bereits erläutert, sowohl unmittelbar innerhalb des Messvolumens gestreutes Licht als auch von der Reflexionseinheit 17 reflektiertes Streulicht auf. Die Detektionseinheit 15 umfasst eine Korrekturzylinderlinse 151 zur Korrektur einer optischen Wirkung des Glasrohrs 14, eine Kollimatoroptik 157, 152, einen Langpassfilter 153 und eine Fokussieroptik 154.

Der Langpassfilter 153 weist eine Cut-On-Wellenlänge von 650 nm auf. Eine Intensität von Streulichtwellenlängen, die kürzer sind als 650 nm, wird durch den Langpassfilter 153 reduziert. Die Cut-On-Wellenlänge von 650 nm entspricht einer unteren Grenze des Stokes-Raman-Spektrums, das, wie nachfolgend noch im Detail beschrieben wird, mit dem Spektrometer erfasst wird. Insbesondere ein Teil des elastisch gestreuten Lichts der Anregungswellenlänge von 635 nm wird durch den Langpassfilter 153 unterdrückt, wodurch unerwünschte Störungen bei der Erfassung des inelastischen Streulichtspektrums im Spektrometer 18 reduziert werden.

Die Detektionsoptik 15 fokussiert das empfangene Streulicht in einen Fokuspunkt 155, der gleichzeitig den Eingang einer Lichtleitfaser 156 darstellt. Über die Lichtleitfaser 156 wird das Streulicht vom Fokuspunkt 155 aus zum Spektrometer 18 weitergeleitet.

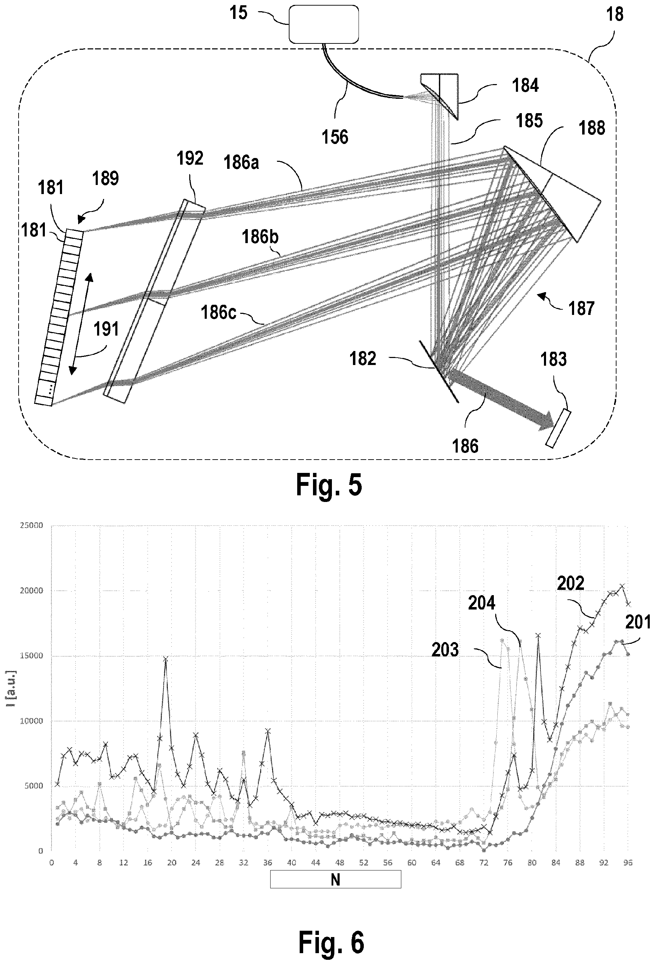

Figur 5 zeigt eine schematische Darstellung des Spektrometers 18 der erfindungsgemäßen Anordnung in einem größeren Detailgrad. Das Streulicht, das von der Detektionsoptik 15 in die Glasfaser 156 fokussiert wird, tritt am Ende der Glasfaser 156 aus und fällt mit einer großen numerischen Apertur von NA= 0,22 in einen kollimierenden Parabolspiegel 184. Dieser kollimiert das Licht zu einem parallelen Strahlenbündel 185, das auf ein optisches Gitter 182 geleitet wird. Am optischen Gitter 182 wird das Strahlenbündel 185 in ein Beugungsmaximum nullter Ordnung 186 und ein Beugungsmaximum erster Ordnung 187 aufgespalten. Das Beugungsmaximum nullter Ordnung 186 enthält innerhalb eines kleinen Beugungswinkelbereichs sämtliche Wellenlängen, die auch im Strahlenbündel 185 enthalten sind. Das Spektrometer umfasst einen Intensitätssensor 183 zur Erfassung einer Intensität des Beugungsmaximums nullter Ordnung 186. Die erfasste Intensität wird - wie nachfolgend noch näher erläutert - zur Ermittlung einer Partikelgröße verwendet.

Aufgrund der am optischen Gitter auftretenden Interferenzeffekte werden innerhalb des Beugungsmaximums erster Ordnung 187 verschiedene Wellenlängenbereiche in unterschiedliche Winkelbereiche aufgefächert. Das nach Wellenlängen aufgefächerte Beugungsmaximum erster Ordnung 187 trifft auf einen fokussierenden Spiegel 188, der den Strahlengang wellenlängenabhängig auf verschiedene Bereiche eines Detektors 189 fokussiert. Der Detektor 189 umfasst eine Anzahl von 96 entlang einer Transversalrichtung 191 nebeneinander positionierten Detektorchips 181. Der Übersicht halber sind in Figur 5 lediglich 20 Detektorchips 181 illustriert, wobei durch das Zeichen "..." angedeutet ist, dass die tatsächliche Anzahl von Detektorchips höher ist. Zudem sind der Übersicht halber lediglich drei verschiedene Wellenlängenbereiche 186a, 186b, 186c des Strahlengangs illustriert, die bei Wellenlängen von etwa 817 nm (186a), 733 nm (186b) und 650 nm (186c) liegen. Die Wellenlängenbereiche 186a, 186b, 186c werden auf verschiedene der Detektorchips 181 fokussiert. Zwischen den in Figur 5 eingezeichneten Wellenlängenbereichen kann das Spektrum des inelastischen Streulichts weitere Wellenlängenbereiche in unterschiedlicher Intensität aufweisen, die mit der Mehrzahl von Detektorchips 181 erfasst werden können. Vor dem Detektor 189 befindet sich ein Korrekturzylinder 192 zur Korrektur eines Astigmatismusfehlers.

Bei den Detektorchips 181 handelt es sich um sogenannte Multipixel Photon Counter (MPPC), wie sie beispielsweise von der Firma Hamamatsu Photonics K.K. erhältlich sind. Jeder Detektorchip 181 ist quadratisch mit einer Seitenlänge von 1 mm ausgebildet und umfasst eine Anzahl von etwa 4356 Lawinen-Dioden, die an einen gemeinsamen Signalausgang des jeweiligen Detektorchips 181 verbunden sind. Eine Lawinen-Diode hat eine Seitenlänge von etwa 15 µm. Trifft ein Photon auf eine einzelne Lawinen-Diode, gibt diese aufgrund des "Lawineneffekts" eine Anzahl von bis zu 10⁸ Elektronen an den Signalausgang weiter. Sämtliche am Signalausgang eines Detektorchips 181 ankommenden Elektronen werden über eine sehr kurze Integrationszeit von lediglich 10 ms gesammelt und auf diese Weise ein Intensitätssignal des Detektorchips 181 erfasst.

Figur 6 zeigt insgesamt vier beispielhafte inelastische Streulichtspektren, die mit Hilfe des in Figur 5 gezeigten Spektrometers 18 aufgenommen wurden. Aufgetragen ist eine gemessene Intensität I in einer beliebigen Einheit gegen die Nummer N des Detektorchips. Erfasstes Streulicht, das auf den Detektorchip mit der Nummer N = 1 auftrifft, weist eine Ramanverschiebung von etwa 350 1/cm auf, was wiederum einer Wellenlänge des inelastischen Streulichts von etwa 650 nm entspricht. Auf den Detektorchip mit der Nummer N = 96 fallendes Streulicht weist eine Ramanverschiebung von etwa 3500 1/cm, was einer Wellenlänge von etwa 817 nm entspricht. Das Spektrum 201 wurde aufgenommen, während sich innerhalb des Messvolumens keine Partikel, sondern lediglich das durch das Glasrohr strömende Wasser befand. Die Spektren 202, 203 und 204 stammen in dieser Reihenfolge aus Messungen an Partikeln aus Polystyrol (PS), Polyethylen (PE) und Polymethylmethacrylat (PMMA). Es zeigt sich, dass die verschiedenen Materialien jeweils ein spezifisches inelastisches Streulichtspektrum generieren, das eine Identifizierung des jeweiligen Materials zulässt.

Zur Auswertung der Spektren werden die erfassten Messdaten an die in Figur 1 gezeigte Auswerteeinheit weitergeleitet. Die Auswerteeinheit umfasst ein Modul zur Materialidentifizierung und ein Modul zur Größenbestimmung. Außerdem führt die Auswerteeinheit die Ergebnisse aller Einzelmessungen zu einer Statistik zusammen, die Auskunft über die Zusammensetzung einer Gesamtprobe gibt. Die Module zur Materialidentifizierung sowie zur Größenbestimmung umfassen jeweils ein trainiertes Modell eines neuronalen Netzes. Das neuronale Netz zur Materialidentifizierung nutzt als Messgrößen die wellenlängenabhängigen Lichtintensitäten des inelastischen Streulichts (Figur 6), das von der gemessenen Probe ausgestrahlt wird. Die Intensitäts-Peaks in dem Spektrum sind in ihrer Position und den Höhenverhältnissen untereinander eindeutig materialspezifisch und erlauben eine chemische Charakterisierung des Materials. Durch die Aufnahme der Materialspektren von bekannten Proben kann eine Datenbasis erzeugt werden, anhand der das neuronale Netz trainiert wird. Auf dieser erlernten Datenbasis kann das neuronale Netz in Echtzeit eine unbekannte Probe (gemessener Einzelpartikel) einem Material zuordnen. Wurde ein Material identifiziert, wird der Datensatz an das Modul zur Größenbestimmung weitergegeben.

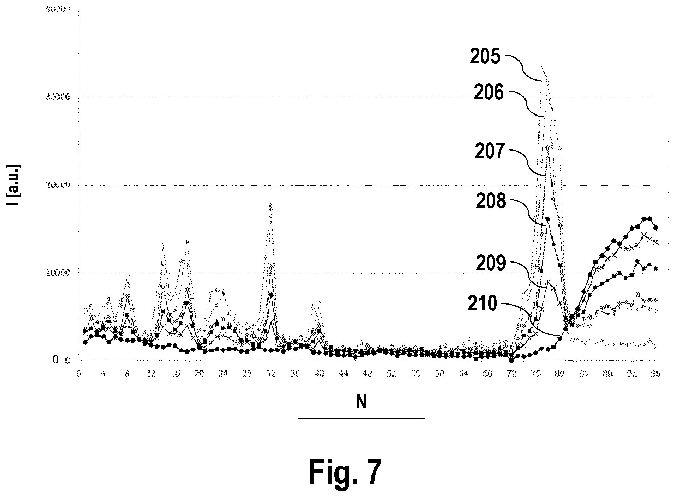

Das neuronale Netz zur Größenbestimmung der Einzelpartikel verwendet als Messgrößen ebenfalls das Raman-Spektrum (inelastisches Streulicht) sowie die vom Sensor 183 erfasste Lichtintensität der nullten Beugungsordnung 186 (Indikator für Menge des elastischen Streulichts). Im Raman-Spektrum des Partikels kann mittels der Intensität des Raman-Peaks von Wasser (siehe Figur 6, Spektrum 201, Peak bei Detektorchip Nummer N = 94 bzw. 3400 1/cm) der im Messvolumen befindliche Wasseranteil abgeschätzt werden. Große Partikel verdrängen das Wasser vollständig aus dem Messvolumen, kleinere nur teilweise, wodurch der Wasserpeak bei hohem Materialanteil im Messvolumen deutlich abfällt. Dies ist in Figur 7 illustriert, in der sechs inelastische Streulichtspektren gezeigt sind, die analog zu den Spektren der Figur 6 an PMMA-Partikeln unterschiedlicher Größe gewonnen wurden. Die Spektren 205 bis 209 zeigen in dieser Reihenfolge Messungen an PMMA-Partikeln, die das Messvolumen etwa zu 100% (205), 95% (206), 75% (207), 50% (208) und 25% (209) ausfüllen. Das Spektrum 210 wurde an einer Wasserprobe ohne darin befindliche Partikel gewonnen (es entspricht insoweit dem Spektrum 201 der Figur 6). Es ist erkennbar, dass der vom Wasser verursachte Peak, der beim Detektorchip mit der Nummer N = 94 (bei einer Verschiebung von etwa 3400 1/cm) liegt, mit der Partikelgröße abnimmt, wodurch die Partikelgröße abgeschätzt werden kann.

Dem neuronalen Netz zur Größenbestimmung kann zusätzlich die vom Sensor 183 gemessene Lichtintensität des elastischen Streulichts in der nullten Beugungsordnung des optischen Gitters zugeführt werden, wodurch die Abschätzung der Partikelgröße weiter verbessert werden kann, da das elastische Streulicht umso intensiver wird, je größer der Partikel ist. Schließlich können dem neuronalen Netz zur Größenbestimmung auch die Messdaten der Photodiode 227 (vgl. Figur 2) zugeführt werden, aus denen ebenfalls Informationen über die Partikelgröße bzw. über die Partikelanzahl gewonnen werden können.

Es kann zudem vorgesehen sein, dass die Auswerteeinheit ein weiteres Modul bestehend aus einem weiteren neuronalen Netz umfasst, um Messergebnisse, die in der Materialidentifizierung nicht erkannt werden konnten, nach ihren Ausprägungen zu gruppieren. Dadurch können gefundene, bisher unbekannte Gruppen durch spätere manuelle Analyse möglicherweise einer weiteren Materialgruppe zugeordnet werden. Diese können dann in der Wissensdatenbank des neuronalen Netzes zur Materialidentifizierung ergänzt und somit durch geringen Aufwand die Identifizierungsmöglichkeiten erweitert werden.

## Patentansprüche

1. Anordnung zur Vermessung von in einer Flüssigkeit mitgeführten Partikeln (20), insbesondere Mikroplastikpartikeln, umfassend:
einen Messkanal (14) mit einem Eingang zum Zuführen der Flüssigkeit;
eine Beleuchtungseinheit (13) zur Aussendung eines Messstrahlengangs (16), der auf ein im Messkanal (14) befindliches Messvolumen (19) der Flüssigkeit auftrifft und wenigstens einen im Messvolumen (19) befindlichen Partikel (20) zur Aussendung von Streulicht anregt;
ein Spektrometer (18) zur Erfassung eines inelastischen Streulichtspektrums des ausgesendeten Streulichts;
eine Detektionsoptik (15), die dazu eingerichtet ist, Anteile (16a, 16b) des Streulichts, die entlang einer Detektionsrichtung auf die Detektionsoptik (15) treffen, zum Spektrometer (18) weiterzuleiten; und
eine Auswerteeinheit (21) zur Identifizierung wenigstens einer Materialart des im Messvolumen (19) befindlichen Partikels (20) anhand des vom Spektrometer (18) aufgenommenen Streulichtspektrums, **dadurch gekennzeichnet, dass**
ein Winkel zwischen einer optischen Achse des Messstrahlengangs (16) und der Detektionsrichtung in einem Bereich zwischen 45° und 135° liegt.

2. Anordnung nach Anspruch 1, bei der der Winkel zwischen der optischen Achse des Messstrahlengangs (16) und der Detektionsrichtung in einem Bereich zwischen 60° und 120°, weiter vorzugsweise zwischen 75° und 105° liegt.

3. Anordnung nach Anspruch 1 oder 2, bei der die auf die Detektionsoptik (15) auftreffenden Anteile (16a, 16b) des Streulichts einen ersten Anteil (16a) des Streulichts umfassen, der ausgehend vom Partikel (20) auf direktem Weg auf die Detektionsoptik (15) trifft;
wobei die Anordnung weiterhin eine Reflexionsoptik (17) umfasst, die dazu eingerichtet ist, einen zweiten vom ersten verschiedenen Anteil (16b) des Streulichts durch Reflexion entlang der Detektionsrichtung zur Detektionsoptik (15) umzulenken, so dass die Detektionsoptik (15) den zweiten Anteil (16b) des Streulichts zum Spektrometer (18) weiterleitet.

4. Anordnung nach einem der Ansprüche 1 bis 3, bei der das Spektrometer einen Detektor (189) mit einer Mehrzahl von entlang einer Transversalrichtung (191) nebeneinander positionierten Detektorchips (181) aufweist,
wobei das Spektrometer (18) dazu eingerichtet ist, verschiedene Wellenlängenbereiche des empfangenen Streulichts in unterschiedliche Winkelbereiche aufzufächern, so dass die verschiedenen Wellenlängenbereiche auf verschiedene der Mehrzahl von Detektorchips (181) gelenkt werden,
wobei jeder Detektorchip (181) einen Signalausgang und jeweils eine Vielzahl von mit dem Signalausgang verbundenen Photoelektronenvervielfacher-Zellen, insbesondere Lawinenphotodioden, aufweist,
wobei die Vielzahl von mit dem Signalausgang verbundenen Photoelektronenvervielfacher-Zellen durch eine Anzahl von Photoelektronenvervielfacher-Zellen gebildet ist, die im Bereich zwischen 1000 und 20000, vorzugsweise zwischen 2000 und 15000, weiter vorzugsweise zwischen 3000 und 10000 liegt.

5. Anordnung nach Anspruch 4, bei der die Mehrzahl von entlang der Transversalrichtung nebeneinander positionierten Detektorchips (181) durch eine Anzahl von Detektorchips (181) gebildet ist, die im Bereich zwischen 30 und 300, vorzugsweise zwischen 40 und 200, weiter vorzugsweise zwischen 50 und 150 liegt.

6. Anordnung nach einem der Ansprüche 4 oder 5, die weiterhin wenigstens eines der nachfolgenden Merkmale aufweist:
eine in Richtung der Transversalrichtung gemessene Seitenlänge der Detektorchips (181) liegt zwischen 0,2 mm und 5 mm, vorzugsweise zwischen 0,4 mm und 3 mm, weiter vorzugsweise zwischen 0,6 mm und 1,5 mm, und/oder
die Detektorchips (181) weisen eine senkrecht zur Seitenlänge gemessene Höhe auf, die größer oder gleich das 0,5-fache und kleiner oder gleich das 2-fache der Seitenlänge der Detektorchips (181), vorzugsweise größer oder gleich das 0,7-fache und kleiner oder gleich das 1,3-fache der Seitenlänge der Detektorchips (181) beträgt.

7. Anordnung nach einem der Ansprüche 1 bis 6, die weiterhin eine Einrichtung (183) zur Erfassung einer Intensität des von der Detektionsoptik (15) an das Spektrometer (18) weitergeleiteten Streulichts umfasst,
wobei die Auswerteeinheit (21) dazu ausgebildet ist, anhand der Intensität des Streulichts eine Partikelgröße zu ermitteln.

8. Anordnung nach Anspruch 7, bei der das Spektrometer (18) ein optisches Gitter (182) umfasst,
wobei die Intensität des Streulichts anhand einer nullten Beugungsordnung des optischen Gitters (182) erfasst wird,
wobei das inelastische Streulichtspektrum anhand einer Beugungsordnung des optischen Gitters (182) erfasst wird, die höher ist als die nullte Beugungsordnung.

9. Anordnung nach einem der Ansprüche 1 bis 8, bei der die Auswerteeinheit dazu ausgebildet ist, eine Partikelgröße des im Messvolumen (19) befindlichen Partikels (20) anhand des inelastischen Streulichtspektrums zu bestimmen, indem das inelastische Streulichtspektrum verglichen wird mit einem inelastischen Streulichtspektrum, das gewonnen wurde, während das Messvolumen (19) frei von Partikeln ist.

10. Anordnung nach einem der Ansprüche 1 bis 9, die weiterhin einen Wellenlängenfilter zur Reduzierung einer Intensität des elastischen Streulichts aufweist, wobei der Wellenlängenfilter vorzugsweise Teil der Detektionsoptik ist.

11. Anordnung nach einem der Ansprüche 1 bis 10, **dadurch gekennzeichnet, dass** die Anordnung weiterhin aufweist:
eine Transmissionseinheit (22) zur Erfassung einer durch das Messvolumen hindurchtretenden Transmission des Messstrahlengangs,
wobei die Auswerteeinheit (21) dazu eingerichtet ist, anhand der Transmission eine Anzahl und/oder eine Abmessung von im Messvolumen befindlichen Partikeln (20) zu bestimmen und/oder festzustellen, ob ein im Messvolumen befindlicher Partikel (20) sich vollständig innerhalb des Messvolumens befindet oder sich bis zu einem Rand des Messvolumens erstreckt.

12. Anordnung nach einem der Ansprüche 1 bis 11, die weiterhin wenigstens eines der folgenden Merkmale aufweist:
der Messstrahlengang weist eine mittlere Strahlungsleistung auf, die größer als 0,5 W, vorzugsweise größer als 1 W, weiter vorzugsweise größer als 1,4 W ist; und/oder
eine Fokussiereinheit (132, 134, 135), die dazu eingerichtet ist, den Messstrahlengang auf das Messvolumen (19) zu fokussieren, wobei das Messvolumen (19) im Querschnitt des Messstrahlengangs (16) betrachtet einen Durchmesser aufweist, der zwischen 10 µm und 800 pm, vorzugsweise zwischen 20 µm und 400 µm, weiter vorzugsweise zwischen 40 µm und 160 µm liegt.

13. Verfahren zur Vermessung von in einer Flüssigkeit mitgeführten Partikeln (20), insbesondere Mikroplastikpartikeln, umfassend die folgenden Schritte:
Zuführen der Flüssigkeit einem Messkanal (14);
Aussenden eines Messstrahlengangs (16) auf ein im Messkanal (14) befindliches Messvolumen (19) der Flüssigkeit, um wenigstens einen im Messvolumen (19) befindlichen Partikel (20) zur Aussendung von Streulicht anzuregen;
Weiterleiten von Anteilen (16a, 16b) des Streulichts entlang einer Detektionsrichtung zu einem Spektrometer (18);
Erfassen eines inelastischen Streulichtspektrums des zum Spektrometer (18) geleiteten Streulichts mit Hilfe des Spektrometers (18);
Auswerten des erfassten Streulichtspektrums, **dadurch gekennzeichnet, dass**
ein Winkel zwischen einer optischen Achse des Messstrahlengangs (16) und der Detektionsrichtung in einem Bereich zwischen 45° und 135° liegt.

14. Verfahren nach Anspruch 13, bei dem die zu dem Spektrometer (18) weitergeleiteten Anteile (16a, 16b) des Streulichts einen ersten Anteil (16a) des Streulichts umfassen, der ausgehend vom Partikel (20) auf direktem Weg zum Spektrometer (18) geleitet wird, wobei das Verfahren weiterhin umfasst:
Reflektieren eines zweiten vom ersten verschiedenen Anteils (16b) des Streulichts, um den zweiten Anteil (16b) entlang der Detektionsrichtung zum Spektrometer (18) weiterzuleiten.

15. Verfahren nach Anspruch 13 oder 14, **dadurch gekennzeichnet, dass** die Flüssigkeit mit einem Volumenstrom durch den Messkanal geleitet wird, der zwischen 2 ml/min und 200 ml/min, vorzugsweise zwischen 5 ml/min und 100 ml/min, weiter vorzugsweise zwischen 10 ml/min und 50 ml/min liegt,
wobei eine für den Durchfluss zur Verfügung stehende Querschnittsfläche des Messkanals vorzugsweise zwischen 7 mm² und 200 mm², weiter vorzugsweise zwischen 20 mm² und 60 mm² liegt.
